# NOUVEAU FASCICULE DE BREVET EUROPEEN

(11) **EP 0 341 103 B2**
(45) Date de publication et mention de la décision concernant l'opposition: **13.08.2003**
(45) Mention de la délivrance du brevet: 16.06.1993
(21) Numéro de dépôt: 89400947.1
(22) Date de dépôt: 06.04.1989
(51) Int. Cl.: A61L 15/32, C07K 14/765

(54) **Procédé de stabilisation des solutions d'albumine humaine et solution obtenue**
Verfahren zum Stabilisieren von menschlichen Albuminlösungen und so erhaltene Lösung
Method for stabilizing solutions of human albumin, and solution so obtained

(30) Priorité: 14.04.1988 FR 8804936
(43) Date de publication de la demande: 08.11.1989
(73) Titulaire: Aventis Pasteur, 69367 Lyon Cedex 07 (FR)
(72) Inventeur: Grandgeorge, Michel, F-69670 Vaugneray (FR); Fournier, Pierre, F-69005 Lyon (FR)
(74) Mandataire: Bernasconi, Jean

(56) Documents cités:
- EP-A- 0 050 061
- EP-A- 0 124 044
- EP-A- 0 131 740
- Pharmacopée européenne (édition 1990), "Albumini Humani Solutio", pages 255 à 255-6
- Pharmacopée européenne (2ème édition II, 1984), "Albumini Humani Solutio", pages 15-20

## Description

L'invention concerne un procédé de stabilisation des solutions d'albumine humaine à usage thérapeutique en vue de leur traitement$ à la chaleur dans un récipient, notamment dans le récipient final.

L'invention concerne également la solution d'albumine obtenue par le procédé conforme à l'invention.

Lors de la fabrication des solutions d'albumine humaine, une étape obligatoire est la pasteurisation finale du produit à 60°C pendant 10 h. Cette étape de chauffage a été introduite aux USA au début des années 1950 afin d'inactiver le virus de l'hépatite B éventuellement présent dans le produit final, malgré les diverses étapes de purification, du fait de la contamination possible de la matière première biologique, en l'occurrence à cette époque, le sang humain. L'efficacité de cette pasteurisation pour éliminer ou réduire le risque de transmission de l'hépatite B par les solutions d'albumine a été démontrée à l'origine chez des volontaires sains. Des études réalisées chez le chimpanzé ont montré ensuite que le virus de l'hépatite non A-non B est également inactivé par un traitement de 10 h à 60°C. Ces données ont fait l'objet d'une revue par GERETY et ARONSON (1). Depuis, diverses études in vitro à l'aide de virus modèles ont démontré que la pasteurisation des solutions protéiques pendant 10 h à 60°C peut être considérée comme une méthode assez générale pour réduire le risque de transmission virale chez le receveur bien que certains virus, comme le parvovirus, soient plus résistants que d'autres à l'inactivation thermique (2-3).

Le brevet EP-A-0.050.061 prévoit une autre solution consistant, dans des préparations de protéines plasmatiques, par exemple de l'albumine, à éviter la pasteurisation par élévation de température et au contraire à inactiver le virus de l'hépatite, et à éliminer éventuellement les endotoxines, par l'action d'agents surfactifs à concentration élevée. Cette solution ne présente cependant pas les avantages de l'inactivation des préparations de l'albumine à la chaleur et nécessite en outre, lorsque la préparation est destinée à l'administration corporelle, éliminer les fortes concentrations d'agent surfactif utilisés à titre d'inactivant.

Le brevet EP-A-0.124.044 décrit un procédé de pasteurisation d'une protéine telle que la fibronectine en présence d'un agent stabilisant adapté à la fibronectine telle que des polyols et notamment un disaccharide auquel il ajoute, dans le but de contrecarrer une dégradation due à l'agitation mécanique de la préparation industrielle de fibronectine pendant le traitement thermique, un agent surfactif et un agent chélateur ou antioxydant. L'ensemble de ce procédé est spécifique à la fibronectine et au traitement de pasteurisation en grand volume à l'échelle industrielle avec agitation mécanique.

Aujourd'hui, l'ensemble des pharmacopées nationales et internationales impose la pasteurisation des solutions d'albumine à 60°C pendant 10 h, cette pasteurisation devant s'effectuer au tout dernier stade de la fabrication, à savoir dans le récipient final, généralement un flacon de verre neutre ; voir par exemple la Pharmacopée Européenne, llème édition, de 1984, la Pharmacopée des USA, XXIème édition et la Pharmacopée Japonaise de 1986. Malgré la relative stabilité de l'albumine à la chaleur, celle-ci doit être protégée, pour éviter toute gélification lors de la pasteurisation, à l'aide de stabilisants appropriés. Les stabilisants utilisés aujourd'hui de manière quasi générale sont le caprylate de sodium et l'acétyle tryptophanate de sodium, seuls ou en combinaison. En particulier, les Pharmacopées USA XXIème édition et Japonaise 1986 imposent l'acétyle tryptophanate de sodium seul, à la dose de 0,16 millimole par gramme d'albumine, ou bien le mélange acétyle tryptophanate de sodium/caprylate de sodium chacun à la dose de 0,08 millimole par gramme d'albumine. Le mandelate de sodium peut également être utilisé seul ou en association avec le caprylate de sodium (voir la Pharmacopée Française VIIIème édition de 1965).

Les stabilisants sont ajoutés en large excès par rapport à l'albumine, à savoir 10 molécules de stabilisant pour 1 molécule d'albumine selon les Pharmacopées USA et japonaise, et du fait de leur affinité propre pour l'albumine, ils sont capables de la protéger efficacement contre une dénaturation directe par la chaleur. En l'absence de ces stabilisants, la dénaturation de l'albumine est inévitable et conduit à une agrégation progressive des molécules d'albumine, ce qui se traduit par l'apparition d'une opalescence puis par une gélification complète de la solution.

Si cette opalescence et cette gélification sont bien évitées par l'emploi des stabilisants décrits précédemment, il est connu qu'après la pasteurisation, un certain nombre de flacons d'albumine présente, lors d'une inspection visuelle, de fines particules floconneuses ou filaments ou peaux en plus ou moins grand nombre. Ce phénomène est plus accentué pour les préparations d'albumine les plus diluées, c'est-à-dire à 4 % ou 5 % de protéines, que pour les préparations les plus concentrées, c'est-à-dire à 20 % de protéines.

Outre que la détection de ces particules insolubles impose le rejet des flacons concernés lors de l'inspection finale dans le cadre du contrôle de qualité, ce qui se traduit par une partie économique, l'apparition de ces particules lors de la pasteurisation traduit une certaine dénaturation du produit, ce qui peut laisser un doute sur sa bonne tolérance lors de l'administration éventuelle à l'homme.

L'invention vise à remédier à cet inconvénient, en proposant un procédé de stabilisation des solutions d'albumine humaine, en vue de leur traitement à la chaleur dans un récipient, notamment le récipient final, permettant une stabilisation parfaite et aboutissant à une solution d'albumine compatible avec l'usage normal de ces solutions.

L'invention a pour objet un Rev 1.

L'agent surfactif utilisé dans le procédé de l'invention ne joue pas le même rôle que la formule stabilisante habituelle et ne peut donc pas s'y substituer. En effet, l'apparition des particules du fait de la pasteurisation ne résulte pas des mêmes causes qui sont à l'origine de l'emploi des stabilisants habituels, comme cela sera illustré plus loin, mais d'interactions de la solution avec la paroi du récipient lors du traitement thermique.

Ce phénomène s'observe dans les flacons de verre neutre classiques, par exemple verre de type I borosilicaté ou verre de type II neutralisé en surface par traitement à l'anhydride sulfureux ou sulfate d'ammonium tels que décrits dans les Pharmacopées Européennes et USA (Société SAINT-GOBAIN DESJONQUERES), mais aussi dans des flacons de matière plastique, par exemple en polystyrène (Société CORNING). Ce phénomène fait donc vraisemblablement intervenir des interactions hydrophobes dénaturantes entre le produit et la paroi du flacon.

Par solution stabilisante habituelle, on entend notamment le caprylate de sodium, l'acétyle tryptophanate de sodium, le mandelate de sodium ou un mélange de deux ou trois de ceux-ci.

Selon l'invention, les concentrations sont avantageusement comprises entre 5 et 50 mg/l de solution à stabiliser. Il suffit d'environ 0,25 à 1 molécule d'un agent surfactif approprié pour 100 molécules d'albumine respectivement en solution de 200 à 50 g/l, pour empêcher la dénaturation et l'apparition de particules insolubles après pasteurisation, en présence de la formule stabilisante habituelle.

L'agent surfactif préféré est le Tween 80 de poids moléculaire moyen de 1320 Dalton. Il doit être utilisé à une concentration supérieure à 5 mg/l et de préférence entre 10 et 20 mg/l. Une concentration de Tween 80 de 10 mg/l de solution à stabiliser, pour une solution d'albumine à 50 g/l, correspond à 1 molécule de ce stabilisant pour 100 molécules d'albumine.

Dans un mode de réalisation particulier de l'invention, l'agent surfactif est ajouté à un stade précoce de la fabrication de l'albumine. En pareil cas, la concentration initiale en agent surfactif doit être telle que le taux résiduel de ce dernier, au moment de la pasteurisation, soit suffisant pour assurer l'effet protecteur désiré.

Les solutions d'albumine humaine concernées par l'invention sont notamment toutes les solutions protéiques dont l'albumine est le composant protéique majoritaire, c'est-à-dire représente plus de 80 % de l'ensemble des protéines, et qui sont destinées à être utilisées en clinique humaine. Elles sont définies, en particulier, dans la Pharmacopée Européenne llème édition de 1984 sous l'appellation "Albumini humani solutio" et dans le Code of Federal Regulations des USA, édition du 1er avril 1986, sous l'appellation "Albumin (human)" et "Plasma protein fraction (human)".

L'albumine humaine concernée par l'invention est notamment obtenue par extraction et purification selon tout procédé convenable à partir d'une source d'albumine humaine ou même, culture de cellules animales, végétales, de bactéries ou de levures transformées pour produire de l'albumine humaine à l'aide des techniques de génie génétique. Parmi les procédés convenables pour l'extraction et la purification de l'albumine humaine, on peut citer en particulier le fractionnement du plasma à l'aide d'alcool décrit par COHN et al. (4) ou le fractionnement du sang placentaire à l'aide d'alcool et de zinc décrit par TAYLOR et al. (5) ou le fractionnement du sang placentaire à l'aide d'alcool, de caprylate de sodium et de gel d'alumine décrit par LIAUTAUD et al. (6) ou le fractionnement du sang placentaire à l'aide d'alcool et par chromatographie tel que décrit par TAYOT et al. (7) ou le fractionnement du plasma par chromatographie tel que décrit dans le brevet USA n° 4.675.384.

L'invention va maintenant être illustrée à l'aide d'exemples non limitatifs.

### EXEMPLE 1

On a essayé d'améliorer la stabilité d'une solution d'albumine à 50 g/l, obtenue par fractionnement de sang placentaire à l'aide d'alcool et par chromatographie comme décrit par TAYOT et al. (4). L'albumine obtenue a une pureté protéique de 100 % en analyse par électrophorèse sur acétate de cellulose et en immuno-électrophorèse bidimensionnelle (présence d'un pic unique).

L'essai a consisté à augmenter les quantités de stabilisants classiques caprylate de sodium et acétyle tryptophanate de sodium par rapport aux quantités imposées par la Pharmacopée USA tout en maintenant un taux de sodium de 145 meq/l et un pH de 7,0. L'albumine a été filtrée puis répartie en flacons de verre de type I de 100 ml et pasteurisée 10 h à 60°C en bain-marie. Les flacons ont été inspectés avant et après pasteurisation et refroidissement spontané à l'air. Aucun flacon ne présentait de particule visible avant la pasteurisation.

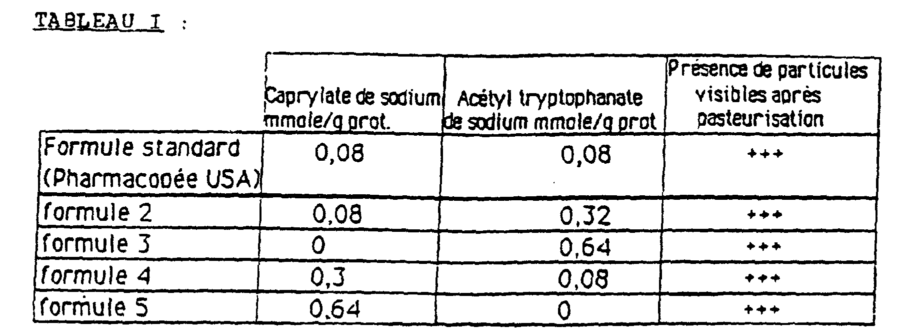

On constate (Voir tableau I) que les stabilisants classiques, malgré des doses fortement augmentées, ne permettent pas d'éviter la présence de particules dans les flacons pasteurisés. Ce constat confirme le fait que la gélrfication empêchée par les stabilisants classiques et la formation de particules sont deux phénomènes physiques indépendants.

### EXEMPLE 2

A partir d'une albumine placentaire préparée comme dans l'exemple 1, on a préparé des solutions à 200 g/l, 50 g/l et 10 g/l de protéines stabilisées avec une formule stabilisante standard, soit 0,08 mmole de caprylate de sodium/g de protéine et 0,08 mmole d'acétyle tryptophanate de sodium/g de protéine. Le sodium a été ajusté à 145 meq/l avec du chlorure de sodium et le pH a été ajusté à 7,0. Les solutions ont été ensuite additionnées de 0 ou 5 ou 10 mg/l de Tween 80, puis filtrées et réparties en flacons de verre de type I de 100 ml, puis pasteurisées 10 h à 60°C en bain-marie. Aucun flacon ne présentait de particule visible avant pasteurisation.

**TABLEAU II :**

| Concentration en albumine (g/l) | 10 | | | 50 | | | 200 | | |
|---|---|---|---|---|---|---|---|---|---|
| Taux de Tween 80 (mg/l) | 0 | 5 | 10 | 0 | 5 | 10 | 0 | 5 | 10 |
| Présence de particules après pasteurisation | +++ | + | 0 | +++ | + | 0 | ++ | + | 0 |

On constate (Voir tableau II) que le Tween 80 empêche totalement la formation de particules visibles à la dose de 10 mg/l quelle que soit la concentration en albumine dans la solution.

### EXEMPLE 3

On a essayé d'améliorer la stabilité d'une solution d'albumine à 50 g/l obtenue par fractionnement de plasma par la méthode de COHN. L'albumine a été fournie sous forme lyophilisée par la société HYLAND. La poudre a été remise en solution et ajustée à 50 g/l de protéines et stabilisée avec une formule stabilisante standard, soit 0,08 mmole de caprylate de sodium/g de protéine et 0,08 mmole d'acétyle tryptophanate de sodium/g de protéine. Le sodium a été ajusté à 145 meq/l avec du chlorure de sodium et le pH a été ajusté à 7,0. Une partie de cette solution a été additionnée de 10 mg/l de Tween 80. Les deux solutions obtenues ont été filtrées et réparties en flacons de verre de type I de 100 ml puis pasteurisées à 60°C pendant 10 h en bain-marie. Aucun flacon ne présentait de particule visible avant pasteurisation.

**TABLEAU III :**

| | | |
|---|---|---|
| Taux de Tween 80 (mg/l) | 0 | 10 |
| Présence de particules visibles après pasteurisation | +++ | 0 |

On constate (voir tableau III) que le Tween 80 à la dose de 10 mg/l empêche totalement la formation de particules visibles dans l'albumine de COHN à 50 g/l soumise à une pasteurisation de 10 h à 60°C.

## Revendications

1. Procédé de traitement par pasteurisation à la chaleur d'une solution d'albumine humaine pure au stade défini par la Pharmacopée Européenne (Ilème édition de 1964 sous l'appellation « Albumini humani solutio ») ou le Code of Federal Regulations (Edition du 1^{er} avril 1986 sous l'appellation « Albumin (human) et « plasma protein fraction (human)) dans lequel elle peut être répartie dans le flacon final pour son usage thérapeutique, en présence d'un agent stabilisant adapté à la stabilisation de l'albumine pendant son traitement à la chaleur, **caractérisé en ce que** l'on effectue ledit traitement à la chaleur dans un récipient en présence, en plus dudit agent stabilisant, d'un agent surfactif choisi parmi le Tween 80, le Tween 20, le Pluronic F68, et le laurate de polyéthylène glycol 600, ledit traitement n'entraînant pas une dénaturation visible.

2. Procédé selon la revendication 1, **caractérisé en ce que** ledit traitement est effectué dans le récipient final de la solution d'albumine.

3. Procédé selon l'une des revendications 1 et 2, **caractérisé en ce que** l'agent surfactif est utilisé à une concentration allant de 5 à 50 mg/l.

4. Procédé selon la revendication 3, **caractérisé en ce que** le Tween 80 est utilisé à une concentration supérieure à 5 mg/l et notamment comprise entre 10 et 20 mg/l.

5. Procédé selon l'une quelconque des revendications 2 à 4, **caractérisé en ce que** l'on ajoute, à la solution d'albumine humaine à stabiliser, la formule stabilisante habituelle, on ajuste le sodium à 145 meq/l avec du chlorure de sodium et le pH à 7,0, on ajoute l'agent surfactif, on filtre puis on répartit la solution dans des flacons et l'on pasteurise à 60°C pendant 10h.

6. Procédé selon la revendication 5, **caractérisé en ce que** la formule stabilisante habituelle se compose de 0,08 mmole de caprylate de sodium et 0,08 mmole d'acétyle tryptophanate de sodium par gramme d'albumine.

7. Procédé selon la revendication 1, **caractérisé en ce qu'**on ajoute l'agent surfactif à un stade précoce de la fabrication de l'albumine.

## Patentansprüche

1. Pasteurisations-Wärmebehandlungsverfahren einer Humanalbumin-Lösung mit einer Reinheit gemäß dem in Pharmacopée Européenne (2. Ausgabe von 1984 unter der Bczeichnung "Albumini humani solutio") oder dem Code of Federal Regulations (Ausgabe vom 1. April 1986 unter der Bezeichnung "Albumin (human)" und "Plasma protein fraction (human)") definierten Zustand, wobei diese in die endgültigen Fläschchen für die therapeutische Verwendung verteilt werden kann, in Gegenwart eines Stabilisierungsmittels, das an die Stabilisierung des Albumins während dessen Wärmebehandlung angepasst ist, **dadurch gekennzeichnet, dass** man die Wärmebehandlung in einem Behälter in Gegenwart eines oberflächenaktiven Mittels, ausgewählt aus Tween 80, Tween 20, Pluronic F68 und Laurat von Polyethylenglycol 600, und weiterhin des Stabilisierungsmittels, durchführt.

2. Verfahren nach Anspruch 1, **dadurch gekennzeichnet, dass** die Behandlung in dem Endbehälter der Albuminlösung durchgeführt wird.

3. Verfahren nach einem der Ansprüche 1 oder 2, **dadurch gekennzeichnet, dass** das oberflächenaktive Mittel mit einer Konzentration von 5 - 50 mg/l verwendet wird.

4. Verfahren nach Anspruch 3, **dadurch gekennzeichnet, dass** das Tween 80 mit einer Konzentration oberhalb 5mg/l, und insbesondere von 10 - 20 mg/l, eingesetzt wird.

5. Verfahren nach einem der Ansprüche 2 bis 4, **dadurch gekennzeichnet, dass** man zu der zu stabilisierenden Humanalbumin-Lösung die übliche stabilisierende Formulierung zugibt, das Natrium mit Natriumchlorid auf 145 mÄq/l und den pH auf 7,0 einstellt, man das oberflächenaktive Mittel zufügt, filtriert, dann die Lösung in Fläschchen verteilt und 10 Stunden bei 60°C pasteurisiert.

6. Verfahren nach Anspruch 5, **dadurch gekennzeichnet, dass** die übliche stabilisierende Formulierung aus 0,08 mMol Natriumcaprylat und 0,08 mMol Natriumacetyltryptophanat pro Gramm Albumin zusammensetzt ist.

7. Verfahren nach Anspruch 1, **dadurch gekennzeichnet, dass** man das oberflächenaktive Mittel in einem frühen Stadium der Herstellung des Albumins zusetzt.

## Claims

1. Process for treating, by heat pasteurization, a solution of pure human albumin at the stage defined by the European Pharmacopoeia (II edition of 1984 under the name "Albumini humani solutio") or the Code of Federal Regulations (Edition of 1st April 1986 under the name "Albumin (human)" and "plasma protein fraction (human)") in which it can be distributed into the final vial for its therapeutic use, in the presence of a stabilizing agent suitable for stabilizing albumin during its heat treatment, **characterized in that** the said heat treatment is carried out in a container in the presence, in addition to the said stabilizing agent, of a surfactant chosen from Tween 80, Tween 20, Pluronic P68, and polyethylene glycol 600 laurate, the said treatment not causing visible denaturation.

2. Process according to Claim 1, **characterized in that** the said treatment is carried out in the final container for the albumin solution.

3. Process according to either of Claims 1 and 2, **characterized in that** the surfactant is used at a concentration ranging from 5 to 50 mg/l.

4. Process according to Claim 3, **characterized in that** Tween 80 is used at a concentration greater than 5 mg/l and especially between 10 and 20 mg/l.

5. Process according to any one of Claims 2 to 4, **characterized in that** the usual stabilizing formula is added to the solution of human albumin to be stabilized, the sodium is adjusted to 145 meq/l with sodium chloride and the pH to 7.0, the surfactant is added, the mixture filtered and then the solution distributed into bottles and pasteurised at 60°C for 10 h.

6. Process according to Claim 5, **characterized in that** the usual stabilizing formula is composed of 0.08 mmol of sodium caprylate and 0.08 mmol of sodium acetyl tryptophanate per gram of albumin.

7. Process according to Claim 1, **characterized in that** the surfactant is added at an early stage of the albumin manufacture.
